Europäisches Patentamt

European Patent Office

Office européen des brevets

(19)

(11) **EP 1 296 140 A1**

(12) **EUROPEAN PATENT APPLICATION**
published in accordance with Art. 158(3) EPC

(43) Date of publication:
26.03.2003 Bulletin 2003/13

(51) Int Cl.⁷: **G01N 33/53**, G01N 33/543,
G01N 33/566

(21) Application number: 01943834.0

(22) Date of filing: 27.06.2001

(86) International application number:
PCT/JP01/05501

(87) International publication number:
WO 02/001224 (03.01.2002 Gazette 2002/01)

(84) Designated Contracting States:
AT BE CH CY DE DK ES FI FR GB GR IE IT LI LU
MC NL PT SE TR
Designated Extension States:
AL LT LV MK RO SI

(30) Priority: 27.06.2000 JP 2000192829

(71) Applicant: CHUGAI SEIYAKU KABUSHIKI
KAISHA
Tokyo, 115-8543 (JP)

(72) Inventors:
• TANAKA, Megumi,
Chugai Seiyaku Kabushiki Kaisha
Gotenba-shi, Shizuoka 412-8543 (JP)

• KAKUTA, Masaya,
Chugai Seiyaku Kabushiki Kaisha
Toshima-ku, Tokyo 171-8545 (JP)
• KOGA, Akiko, Chugai Seiyaku Kabushiki Kaisha
Gotenba-shi, Shizuoka 412-8543 (JP)
• ESAKI, Keiko, Chugai Seiyaku Kabushiki Kaisha
Gotenba-shi, Shizuoka 412-8543 (JP)

(74) Representative: Maschio, Antonio et al
D Young & Co,
21 New Fetter Lane
London EC4A 1DA (GB)

(54) **METHOD OF EVALUATING BINDING ACTIVITY OF LIGAND TO LIGAND-BINDING PROTEIN**

(57)    The present invention provides a convenient and highly accurate method of measuring the biological activity of a ligand. Namely, this method of evaluating the binding activity of a ligand to a ligand-binding protein comprises; measuring the binding activity value of the ligand to the ligand-binding protein, and measuring the binding activity value of the above-described ligand to a second ligand-binding substance binding to a site other than the binding site where the ligand binds to the above-described ligand-binding protein.

EP 1 296 140 A1

**Description**

TECHNICAL FIELD

The present invention relates to a method of evaluating the binding activity of a ligand to a ligand-binding protein.

BACKGROUND ART

To measure antibody-antigen interaction, radioimmunoassay (RIA), enzyme-linked immunosorbent assay (ELISA) and the like have been used. Recently, the use of an optical phenomenon, surface plasmon resonance (hereinafter, referred to as "SPR") has enabled real time monitoring of antibody-antigen interaction without labeling. As an example, a instrument for analyzing antibody-antigen interaction using a SPR sensor, referred to as BIACORE™ is being marketed by BIACORE (formerly Pharmacia Biotech).

The basic system of BIACORE™ consists of a light source, a prism, a detector and microfluidic system. In practice, ligands are immobilized on a cassette-type sensor chip, into which an analyte is injected. If the analyte has affinity for the ligands, the amount of analyte-bound to ligands is detected optically.

The detection principle is a phenomenon referred to as SPR. Specifically, incident light is totally reflected into the interface between a glass and a thin metal film. The incident light at a certain angle excites surface plasmons, thus reducing the reflected light intensity at that angle. This angle is varied depending on changes in the concentration of the solvent that is in contact with the thin metal film (sensor). BIACORE™ detects these changes.

In the BIACORE™ system, this change is referred to as a SPR signal, and a shift in resonance angle of 0.1 degree represents 1,000 RU (resonance units). A response of 1,000 RU corresponds to a change when approximately 1 ng of protein is bound onto a thin metal sensor with a surface area of 1 mm$^2$. BIACORE™ can sufficiently detect changes of about 50 RU (50 pg) for protein.

A computer provided with BIACORE™ converts the detected signals into a binding curve, referred to as a sensorgram, which is drawn on the computer display in real-time (Toru Natsume et al., 1995, Experimental medicine, 13, p563-569) (Karlsson, R. et al., (1991) J. Immunol. Methods 145, p229-240).

Using BIACORE™, the kinetics parameters of antibodies, specifically, the dissociation constant (KD), dissociation rate constant (Kdiss) and association rate constant (Kass) can be measured.

However, kinetics analysis of antigen-antibody reaction using the above-mentioned BIACORE™ is inappropriate for treatment with many analytes, because it is time consuming to study the conditions. Therefore, there is a need for a convenient and highly accurate method capable of measuring the biological activity of antibodies while maintaining the specificity of BIACORE™ and is appropriate for studying the quality design and prescription design of antibodies.

DISCLOSURE OF THE INVENTION

An object of the present invention is to provide a convenient and highly accurate method of measuring the biological activity of a ligand (in particular, an antibody).

The present inventors have studied a method of measuring the binding activity by quantitatively determining the amount of antibodies bound to antigens in order to conveniently evaluate in vitro the biological activity of an antibody without kinetics analysis. As a result, the present inventors have completed the present invention by succeeding in establishing a highly accurate and convenient test method of evaluating the binding activity of antibodies by correcting the antibody concentration by means of a system that quantitatively determines the amount of antibodies bound to Protein A.

Specifically, the present invention provides a method of evaluating the binding activity of a ligand to a ligand-binding protein, which comprises; measuring the binding activity value of a ligand to a ligand-binding protein, and measuring the binding activity value of the ligand to a second ligand-binding substance which binds to a ligand site other than the binding site where the ligand binds to the ligand-binding protein.

Either the ligand or the ligand-binding protein may be immobilized.

The ligand may be preferably an antigen or the antibody thereof, an enzyme or the substrate protein thereof or a ligand for various receptors.

In the above method, the ligand may be an antibody, and the ligand-binding protein may be an antigen peptide.

The second ligand-binding substance may be selected from substances which can specifically bind to sites other than the antigen-binding site, such as the constant regions (an Fc portion, an L chain κ region, an L chain λ region, or the like) and the sugar chain of an antibody. In particular, the second ligand-binding substance may be a substance which specifically binds to the Fc portion of an antibody.

A substance which specifically binds to the Fc portion of an antibody is preferably Protein A, protein G, protein L, Fc receptor or lectin.

In the method of the present invention, FLAG protein is fused to a ligand, and a substance which specifically binds to the FLAG protein may be used as a second ligand-binding protein.

The ligand-binding protein and the second ligand-binding substance may be separately immobilized, and then allowed to react respectively with the same ligand-containing samples, so that each of the binding activities may be measured.

In a preferred aspect of the present invention, the ligand is an antibody, and the ligand-binding protein is an antigen peptide, and the second ligand-binding substance is Protein A.

When the activity of an antibody to an antigen peptide is evaluated by the method of the present invention, the binding activity value of the antibody to the antigen peptide is preferably corrected with the binding activity value of the antibody to the second ligand-binding substance.

For example, the binding activity of an antibody to an antigen peptide can be evaluated using the binding activity ratio of the antibody calculated by the following formula (I):

$$\text{Binding activity ratio of antibody (\%)} = \frac{\text{Binding activity value of antibody to antigen peptide}}{\text{Binding activity value of antibody to second ligand-binding substance}} \times 100 \quad (\text{I})$$

The binding activity value of an antibody to an antigen peptide, and the binding activity value of the antibody to the second ligand-binding substance can be calculated by the following formulae (II) and (III), respectively:

$$\text{Binding activity value of antibody to antigen peptide} = \frac{\text{Binding concentration of antibody of unknown sample to antigen peptide}}{\text{Binding concentration of antibody of standard sample to antigen peptide}} \times 100 \quad (\text{II})$$

$$\text{Binding activity value of antibody to second ligand-binding substance} = \frac{\text{Binding concentration of antibody of unknown sample to second ligand-binding substance}}{\text{Binding concentration of antibody of standard sample to second ligand-binding substance}} \times 100 \quad (\text{III})$$

The binding concentration of an antibody to an antigen peptide, and that of the antibody to a second ligand-binding substance can be measured by SPR, a fluorescence polarization measurement method, a radioimmunoassay (RIA), an enzyme-linked immunosorbent assay (ELISA), HPLC, an analytical ultracentrifugation method or a titration calorimetry. In particular, it is preferable to measure by SPR.

As an example of the above methods, the binding activity of an antibody of an unknown sample to an antigen can be evaluated by the binding activity ratio of the antibody calculated by the following formula (I'):

$$\text{Binding activity ratio of antibody (\%)} = \frac{\text{Binding activity value of antibody to antigen peptide}}{\text{Binding activity value of antibody to Protein A}} \times 100 \quad (\text{I'})$$

The binding activity value of an antibody to an antigen, and that of an antibody to Protein A can be calculated by the following formulae (II) and (III'), respectively:

$$\text{Binding activity value of antibody to antigen peptide} = \frac{\text{Binding concentration of antibody of unknown sample to antigen peptide}}{\text{Binding concentration of antibody of standard sample to antigen peptide}} \times 100 \quad (\text{II})$$

$$\text{Binding activity value of antibody to Protein A} = \frac{\text{Binding concentration of antibody of unknown sample to Protein A}}{\text{Binding concentration of antibody of standard sample to Protein A}} \times 100 \quad (\text{III'})$$

The binding concentration of an antibody to an antigen peptide, and that to Protein A can be measured by SPR, a fluorescence polarization measurement method, a radioimmunoassay (RIA), an enzyme-linked immunosorbent assay (ELISA), HPLC, an analytical ultracentrifugation method, a titration calorimetry or the like. Of these, the measurement by SPR is convenient, because it can monitor in real-time the antibody-antigen interaction without labeling.

Target antibodies of the method of the present invention may be antibodies for any antigens. Examples of such an antibody include an antibody (hereinafter, referred to as "anti-PTHrP antibody") for parathyroid hormone-related peptide (hereinafter, referred to as "PTHrP"), an antibody for IL-6 receptor, an antibody for HM1.24 antigen, and an antibody for tissue factor (TF).

The antibody may be either a monoclonal antibody or a polyclonal antibody, and a monoclonal antibody is preferable.

The antibody class may be any of IgM, IgD, IgG, IgA and IgE, and IgG is desirable. In addition, the subclass of H chain may be any of IgG1, IgG2, IgG3, and IgG4, and IgG1 and IgG4 are desirable, and the subclass of L chain may be either κ or λ.

A monoclonal antibody may be a human antibody, humanized antibody or chimeric antibody.

In addition, examples of target substances that can be measured by the measurement method of the present invention also include antibody fragments, such as Fab, $(Fab')_2$ and re-constituted antibody fragments, such as a single-chain Fv.

A preferred antibody forms few (preferably, 5% or less) or no oligomer, such as dimmers.

In the present specification, the term "second ligand-binding substance" indicates a substance which binds to a ligand at a site other than the binding site of the ligand with a ligand-binding protein (also referred to as "first ligand-binding substance"). When a ligand is an antibody, a second ligand-binding substance specifically binds to a portion other than the antigen binding site, such as the constant region (Fc portion, L chain κ region, an L chain λ region, or the like) and the sugar chain of the antibody. Examples of a substance which binds to Fc portion include Protein A, protein G and Fc receptor. Examples of a substance which binds to L chain κ region include protein L and anti-κ antibody. An example of a substance which binds to L chain λ region is anti-λ antibody. Examples of a substance which binds to sugar chains include lectins, such as RCA, LCA and ConA.

Preferred examples of a substance which binds to a portion other than the antigen binding site of an antibody include Protein A, protein G, Fc receptor, protein L and lectins, such as RCA, LCA and ConA. More preferred examples include Protein A, protein G and protein L, and most preferably, Protein A.

In addition, the present invention provides a kit for evaluating the binding activity of a ligand to a ligand-binding protein. This kit contains both a ligand-binding protein and a second ligand-binding substance. This ligand-binding protein may be an antigen peptide. Furthermore, the kit of the present invention may contain other reagents. For example, when SPR is used for measuring the binding activity of an antibody to an antigen, the kit of the present invention may further contain a reagent for immobilizing onto a sensor chip an antigen and a substance which binds to a portion other than the antigen-binding site of an antibody. The reagent may be any reagent as long as it activates the functional group of an antigen, for example, an amino group, thiol group, or aldehyde group to bind onto the chip. Examples of such a reagent include N-ethyl-N'-(3-dimethylaminopropyl) carbodiimidehydrochloride, N-hydroxysuccinimide, ethanolamine hydrochloride, 2-(2-pyridinyldithio) ethaneamine hydrochloride, cysteine, biotinhydrazide.

Even if other protein is present as a contaminant, or a medium, a stabilizer or the like in a sample solution, the method of evaluating ligand (for example, antibody) activity and the kit of the present invention do not require additional purification procedures to evaluate the binding activity ratio of the ligand (for example, antibody) molecule. Therefore, the evaluation method and the kit can be used for studying quality control, in-process control and formulation design of antibodies in the production process or purified antibodies as an drug substance.

In addition, the binding activity ratio which is finally obtained by the method of evaluating ligand (for example, antibody) activity and the kit of the present invention does not reflect the concentration of an active ingredient in a sample solution, but the activity of the binding activity of a ligand (for example, antibody) to a second ligand-binding activity. Therefore, the activity ratio of 100% or more may suggest that some decomposition occurs at a second portion (for example, Fc portion) other than the antigen-binding site of an antibody; and the activity ratio of 100% or less may suggest some decomposition at the antigen-binding site of an antibody. Accordingly, the method and the kit of the present invention in combination with another method of evaluating physical properties can be used for stability testing and shelf life setting.

Furthermore, the method of evaluating ligand (for example, antibody) activity and the kit of the present invention can also be used for selecting a highly active protein (in particular, a mutant protein, various monoclonal antibodies or the like). Specifically, the method and the kit of the present invention are effective in selecting a mutant protein with high biological activity or stability resulting from point mutation, or the like. The method and the kit of the present invention are particularly useful in screening antibodies, which are reconstituted from natural antibodies, such as chimeric antibodies and humanized antibodies.

Further, the method and the kit of the present invention are also effective of evaluating whether or not various electrically charged hetero components (the plural minor peaks, which are detected when the bulk protein, is applied to ion exchange chromatography) detected in the bulk proteins, are "Product-Related Substances," as is required for New Drug Application of a protein drug product, such as an antibody.

Furthermore, the method of evaluating ligand (for example, antibody) binding activity, and the kit of the present invention can provide a convenient and highly accurate measurement of the binding activity of, and the quantitative determination of highly active proteins within body fluids, such as blood, serum or the like (in particular, a blood factor, a mutant protein, various monoclonal antibodies, antibody fragments, such as Fab and $(Fab')_2$, a single-chain Fv and the like).

BRIEF DESCRIPTION OF DRAWINGS

Figure 1 shows a sensorgram when PTHrP(1-34+Cys) was immobilized.
Figure 2 shows a sensorgram (1Cycle) when anti-PTHrP antibody *a* (2.0 μg/mL) was measured.
Figure 3 shows the calibration curve (Duplicate) when the amount of anti-PTHrP antibody bound to PTHrP was quantitatively determined.
Figure 4 shows a cycle ignored from data because of a disturbance in the sensorgram.
Figure 5 shows a sensorgram when Protein A was immobilized.
Figure 6 shows a sensorgram (1Cycle, Protein A) when anti-PTHrP antibody *a* (2.0 μg/mL) was measured.
Figure 7 shows the calibration curve (Duplicate) when the amount of anti-PTHrP antibody bound to Protein A was quantitatively determined.

This specification includes part or all of the contents as disclosed in the specification of Japanese Patent Application No. 2000-192829, which is a priority document of the present application.

BEST MODE FOR CARRYING OUT THE INVENTION

A method of evaluating the binding activity of an anti-PTHrP antibody to PTHrP is described as an example of the method of the present invention. This method comprises the steps of measuring, using BIACORE™, the amount of anti-PTHrP antibody bound to PTHrP and to Protein A respectively, and of correcting the measured amount of the anti-PTHrP antibody bound to PTHrP with the measured amount of the anti-PTHrP antibody bound to Protein A.

First, anti-PTHrP antibodies are produced according to the method described in WO98/13388.

Anti-PTHrP antibodies are diluted with water, and then a solution of the anti-PTHrP antibody is prepared at an appropriate concentration (for example, 100-500 μg/mL). The solution is quantitatively determined by the absorbance method in the general test of the Japanese Pharmacopoeia. The solution is diluted with HBS buffer solution (HBS-EP Buffer (BIACORE)) at an appropriate concentration (for example, 10-50 μg/mL). This preparation is repeated three times. HBS buffer solution is added to each solution, so that solutions each having a certain concentration (for example, 1-5 μg/mL) are prepared as unknown sample solutions 1, 2 and 3.

The above procedure is performed for a standard product of anti-PTHrP antibody, thereby preparing standard sample solutions 1, 2 and 3.

Separately, anti-PTHrP antibodies are diluted with water, and then a solution of anti-PTHrP antibody at an appropriate concentration (for example, 100-500 μg/mL) is prepared. The solution is diluted with HBS buffer solution, and then solutions of anti-PTHrP antibody having various concentrations (for example, 0 to 5 μg/mL) are prepared as standard solutions A, B, C, D, E and F for a calibration curve.

Next, PTHrP- and Protein A-immobilized chips are prepared. A sensor chip CM 5 (BIACORE, Code # BR-1000-14) is docked in BIACORE™, and then HBS buffer solution is run at a flow rate of 5-20 μL/min. Then, different solutions are run successively, to immobilize PTHrP(1-34+Cys) on the chip: for example, 150 μL of a mixed solution containing equal amounts of EDC (N-ethyl-N'-(3-dimethyl aminopropyl)-carbodiimide hydrochloride) solution and NHS (N-hydroxysuccinimide) solution; for example, 150 μL of borate buffer solution of PDEA (2-(2-pyridinyldithio)ethaneaminehydrochloride); for example, 40 μL of acetate buffer solution of PTHrP(1-34+Cys) (a synthetic peptide having Cys added to the C terminus of 1-34 peptides of PTHrP); for example, 150 μL of formate buffer solution of cysteine-sodium chloride; for example, 10 μL of glycine hydrochloride buffer solution; and for example, 10 μL of 10 mM hydrochloric acid aqueous solution. Using another flow cell, HBS buffer solution is run similarly at a flow rate of 5 to 20 μL/min. Then, different solutions are run successively to immobilize Protein A onto a chip: for example, 100 μL of the mixed solution of equal amounts of EDC solution and NHS solution; for example, 40 μL of acetate buffer solution of Protein A; for example, 100 μL of ethanolamine hydrochloride buffer solution; for example, 10 μL of glycine hydrochloride buffer solution; and for example, 10 μL of 10 mM hydrochloric acid aqueous solution.

The standard solutions for a calibration curve A, B, C, D, E and F, the standard sample solutions 1, 2 and 3, and the unknown sample solutions 1, 2 and 3 are applied to the PTHrP-immobilized chip and the Protein A-immobilized chip respectively. Two measurements are performed for each batch. Binding phase was defined two minutes period, 1 to 100 μL, preferably, 10 μL each of the standard sample solutions and the unknown sample solutions are injected. Here, the bound amount is a difference between the resonance unit (RU), provided 20 to 60 seconds after the solution is switched to the HBS buffer solution, and baseline. Subsequently, 15 mM hydrochloric acid solution is injected for washing and regenerating the sensor chips. With 1 cycle of analysis consisting of the binding, washing and regeneration, a sensorgram is obtained, a calibration curve is produced using the attached software, and then each binding concentration is calculated. From the binding concentration, the binding activity ratio (%) is calculated by the following formulae:

$$\text{Binding activity ratio of anti-PTHrP antibody (\%)} = \frac{\text{Binding activity value of anti-PTHrP antibody to PTHrP}}{\text{Binding activity value of anti-PTHrP antibody to Protein A}} \times 100$$

$$\text{Binding activity value of anti-PTHrP antibody to PTHrP} = \frac{\text{Binding concentration of anti-PTHrP antibody of unknown sample to PTHrP-immobilized chip}}{\text{Binding concentration of anti-PTHrP antibody of standard sample to PTHrP-immobilization chip}} \times 100$$

$$\text{Binding activity value of anti-PTHrP antibody to Protein A} = \frac{\text{Binding concentration of anti-PTHrP antibody of unknown sample to Protein A-immobilized chip}}{\text{Binding concentration of anti-PTHrP antibody of standard sample to Protein A-immobilized chip}} \times 100$$

The present invention will be described more specifically by the following examples. These examples are given only for explanation, and are not intended to limit the scope of the present invention.

[Example 1]

I. Experiment materials

Instruments, reagents and samples employed in the examples are as follows:

(1) Instrument

BIACORE™ upgrade: (BIACORE)
Sensor Chip : Sensor Chip CM5 : (BIACORE)
Spectrophotometer: DU-640 (BECKMAN)

(2) Reagent

Running Buffer : HBS buffer BIA Certified (0.01 M HEPES pH 7.4, 0.15 M NaCl, 3 mM EDTA, 0.005% (v/v) Surfactant P20) (BIACORE) Code # BR-1001-88
NHS : 0.05 M N-hydroxysuccinimide, Amine coupling kit (BIACORE) Code # BR-1000-50
EDC: 0.2 M N-ethyl-N'-(3-dimethyl aminopropyl)-carbodiimide hydrochloride, Amine coupling kit (BIACORE) Code # BR-1000-50
Ethanolamine-HCl (pH8.5): Amine coupling kit (BIACORE) Code # BR-1000-50
PDEA: Thiol coupling kit (BIACORE) Code # BR-1000-58
PTHrP(1-34+Cys): product synthesized by SAWADY Technology Co., Ltd. (custom peptide JH 365, 21/11/94, B-CAL 35a.a.)
Protein A: (CAPPEL) Cat # 55832, Lot # 40690
L-Cystein, NaCl, HCl
0.1 M Borate-Na Buffer (pH8.5)
10 mM Acetate-Na Buffer (pH4.0, 5.0)
0.1 M Na-Formate Buffer (pH4.3)
0.1 M Gly-HCl Buffer (pH2.5)

(3) Sample

Anti-PTHrP antibodies *a, b, c, d, e* and *f*: in house (all of them are lots for research)

II. Experiment

(1) Study of quantification of binding activity value of anti-PTHrP antibody toPTHrP(1-34+Cys)-immobilized chip

i) Immobilization of antigen PTHrP(1-34+Cys) to sensor chip

The flow rate was set at 5 µL/min, the reagents were injected according to Table 1, and then antigen PTHrP (1-34+Cys) was immobilized by the thiol coupling method.

Table 1.

| Conditions for immobilizing PTHrP(1-34+Cys) | |
|---|---|
| Reagent | Injection volume |
| NHS/EDC | 100 µL |
| PDEA (5.4 mg/250 µL 0.1 M Borate-Na Buffer (pH 8.5)) | 100 µL |
| PTHrP(1-34+Cys) (10 µg/mL 10 mM Na-Acetate Buffer(pH 5.0)) | 20 µL |
| 50 mM Cys / 1 M NaCl (0.1 M Na-Formate Buffer(pH 4.3) | 100 µL |
| 0.1 M Gly-HCl (pH 2.5) | 10 µL |
| 10 mM HCl | 10 µL |

ii) Measurement of binding activity value of anti-PTHrP antibody to PTHrP to study lot to lot consistency

Using the chips prepared under the conditions of i), the respective amounts of anti-PTHrP antibodies bound to PTHrP for 4 lots (*a*, *b*, *c* and *d*) of anti-PTHrP antibody were measured by the following procedures. Then, differences among the lots were studied. Original solutions of samples *a, b, c* and *d,* were diluted with purified water to prepare about 500 µg/mL solutions, and then the concentrations were determined by the absorbance method. Based on the concentrations, 0, 1, 2, 3, 4 and 5 µg/mL (n=1) solution in HBS for a calibration curve and a 2 µg/mL (n=3) solution in HBS as a control were prepared for the standard sample *a*, and 2 µg/mL (n=3) solution in HBS were prepared for the unknown samples *b, c* and *d* respectively. First, the flow rate was set at 5 µL/min, and binding phase was defined two minutes period while 10 µL of the sample solution was injected. After the sample solution was switched to a running buffer, a difference between resonance unit (RU) after 30 seconds and a baseline immediately before injection was determined as the bound amount. Subsequently, the sensor chips were washed and regenerated by injecting 15 mM HCl. With 1 cycle of analysis consisting of the binding and regeneration, measurement was performed twice for each sample solution, so that the sensorgram and the bound amount were obtained.

(2) Study of quantification of binding activity to protein A-immobilized chip

i) Measurement of binding activity value of anti-PTHrP antibody *e* to PTHrP

Original solutions of the samples (*a* and *e*) of anti-PTHrP antibody were diluted with purified water to prepare about 100 µg/mL solutions, and then the concentrations were determined by the absorbance method. Based on the concentrations, 0, 1, 2, 3, 4 and 5 µg/mL (n=1) solution in HBS for a calibration curve and a 2 µg/mL (n=3) solution in HBS as a control were prepared for standard samples *a*, and 2 µg/mL (n=3) solution in HBS were prepared for unknown samples *e*. In a manner similar to procedure (1), the amount of bound PTHrP was measured.

ii) Immobilization of Protein A to sensor chip

The flow rate was set at 5 µL/min, the reagents were injected according to Table 2, and then Protein A was immobilized by the amine coupling method.

Table 2.

| Immobilization conditions for Protein A | |
|---|---|
| Reagent | Injection volume |
| NHS/EDC | 150 µL |
| Protein A (50 µg/mL 0.1 M Borate-Na Buffer (pH 4.0)) | 50 µL |

Table 2. (continued)

| Immobilization conditions for Protein A | |
| --- | --- |
| Reagent | Injection volume |
| Ethanolamine | 150 µL |
| 0.1 M Gly-HCl (pH 2.5) | 10 µL |
| 10 mM HCl | 10 µL |

iii) Measurement of binding activity ratio of anti-PTHrP antibody *e* to PTHrP/Protein A

Based on the concentrations obtained by the absorbance method, 0, 1, 2, 3, 4 and 5 µg/mL (n=1) solution in HBS for a calibration curve and a 2 µg/mL (n=3) solution in HBS as a control were prepared for the standard sample. Diluted solutions (n=3) were prepared by diluting 1000-fold the original sample solution for unknown sample *e*. In a manner similar to procedure (1), the amount of anti-PTHrP antibody bound to PTHrP was measured. Next, similar measurements were performed for Protein A-immobilized chip, thereby obtaining the sensorgram and the amount of anti-PTHrP antibody bound to Protein A.

(3) Measurement of repeatability of the measurement method

Based on the concentrations obtained by the absorbance method, 0, 1, 2, 3, 4 and 5 µg/mL (n=1) solution in HBS for a calibration curve and a 2 µg/mL (n=3) solution in HBS as a control] were prepared for the standard sample *a*. 2 µg/mL (n=3) solution in HBS were prepared for unknown sample *f*. In a manner similar to (2)-iii), 0 to 5 µg/mL samples for a calibration curve (n=1) and 2 µg/mL unknown sample (n=3) were injected to PTHrP-immobilized chip and Protein A-immobilized chip, respectively. Two measurements of the bound amount were considered as one measurement. The series of procedures starting from the preparation of samples were performed 7-times in total, so that the repeatability was studied.

III. Results and discussions

(1) Study of quantification of binding activity to PTHrP(1-34+Cys)-immobilized chip

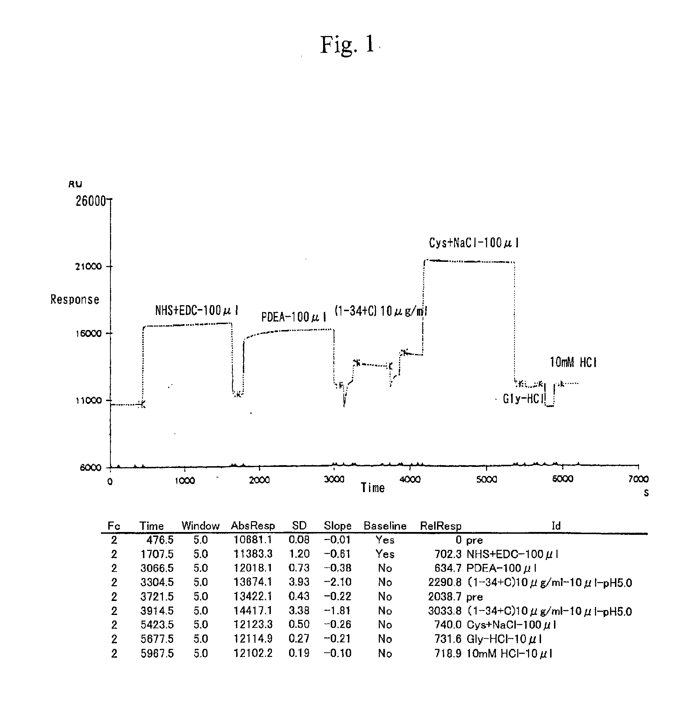

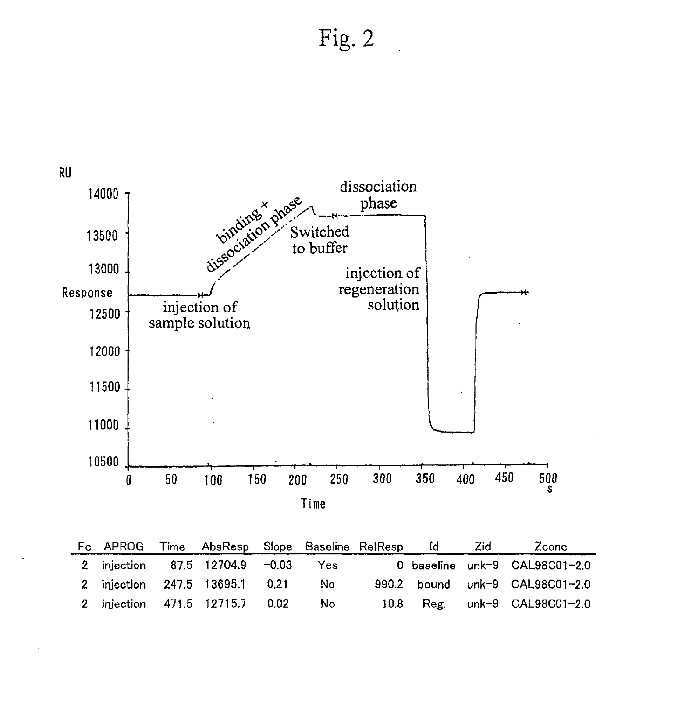

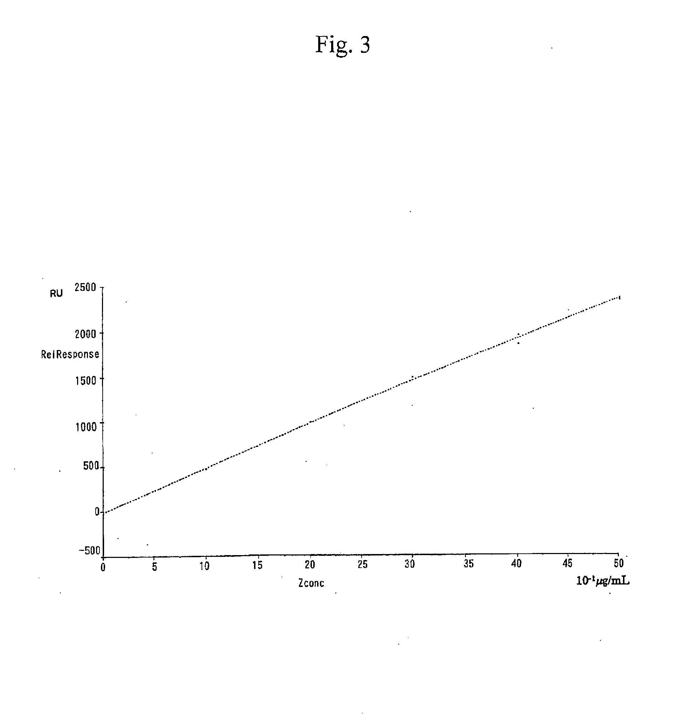

Figure 1 shows the result of immobilization of PTHrP. To evaluate the PTHrP(1-34+Cys)-immobilized chip prepared and to set an appropriate concentration range for a calibration curve and the concentration of a sample, the amount of antibodies bound to PTHrP was measured for 4 lots of anti-PTHrP antibody certified to have approximately equal quality. A representative example of the obtained sensorgram is shown in Fig 2.
A calibration curve was produced from the resulting bound amount using the calculation software attached to the BIACORE™ system for quantitatively determining concentrations (Fig. 3). As a result, the binding phase on the sensorgram (Fig. 2) was almost a straight line, no dissociation was observed, and the calibration curve (Fig. 3) almost maintained its linearity within the concentration range. Taking these results together, it was concluded that the amount of immobilized ligands was sufficient for use in the quantification system. Therefore, the concentrations of samples to quantitatively determine the amount of antibodies bound to a PTHrP(1-34+Cys)-immobilized chip were set according to the conditions employed for this experiment. Specifically, standard samples for a calibration curve were set at six concentrations: 0, 1, 2, 3, 4 and 5 µg/mL, and unknown samples were set at a concentration of 2 µg/mL, which was about middle point of the calibration curve.

ii) Analysis of lot-to-lot consistency

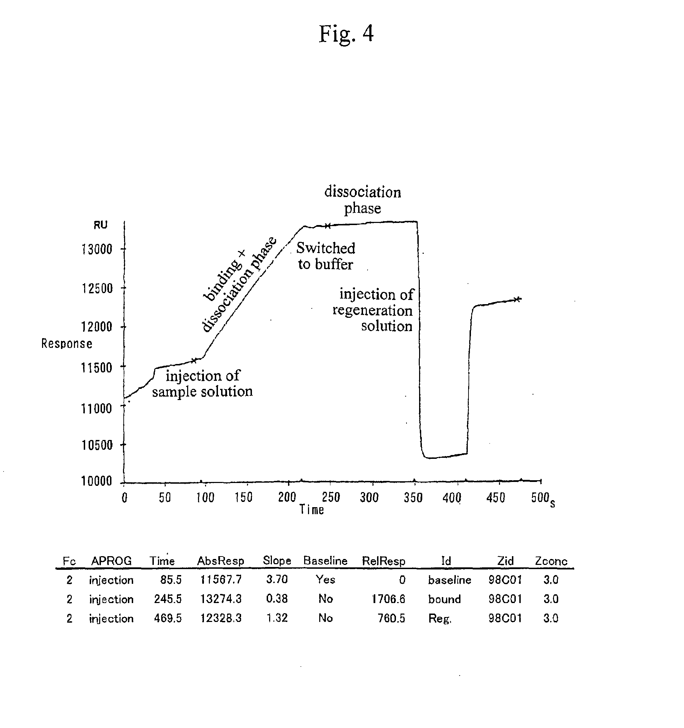

Using the calibration curve, the binding concentrations of 4 lots that were actual samples were calculated with the attached software using the bound amount provided from the sensorgram. At this time, a value was ignored for a cycle for which no appropriate bound amount could be detected due to a disturbance in the sensorgram (Fig. 4). Based on the binding concentrations, PTHrP binding activity value was defined as eq. 1, and then the results of the 4 lots were summarized in Table 3. Thus, the measurement system was shown to be extremely precise such that coefficient of variation was within 3% when n=3. From this experiment, it was concluded that there was no significant difference in the PTHrP binding activity among 4 lots.

$$\text{Binding activity value to PTHrP (BIACORE) (\%)} = \frac{\text{Binding concentration of unknown sample}}{\text{Binding concentration of standard sample}} \times 100 \quad \text{(eq. 1)}$$

Table 3.

| Measurement results of binding activity value to PTHrP (lot to lot consistency) | | | | | | | | | | |
|---|---|---|---|---|---|---|---|---|---|---|
| Sample | n | Bound amount (Resonance unit, RU) | | Binding concentration calculated from calibration curve (μg/mL) | | | | Measurement (measured three times) | | Binding activity value (%) |
| | | 1st. cycle | 2nd. cycle | 1st. cycle | 2nd. cycle | Average | %cv. dupli | Average | %cv | |
| b | 1 | 966.4 | 979.7 | 1.99 | 2.01 | 2.00 | 0.71 | 2.01 | 0.90 | 99.2 |
| | 2 | 979.0 | 957.8 | 2.01 | 1.97 | 1.99 | 1.42 | | | |
| | 3 | 981.4 | 987.2 | 2.02 | 2.03 | 2.03 | 0.35 | | | |
| c | 1 | 951.7 | 1008.7 | 1.96 | 2.07 | 2.02 | 3.86 | 2.05 | 1.73 | 101 |
| | 2 | 987.5 | 985.5 | 2.06 | 2.02 | 2.04 | 1.39 | | | |
| | 3 | 1014.7 | 1019.2 | 2.08 | 2.09 | 2.09 | 0.34 | | | |
| d | 1 | 967.8 | 971.1 | 1.99 | 2.00 | 2.00 | 0.35 | 1.99 | 2.06 | 98.2 |
| | 2 | 974.2 | 913.9 | 2.00 | 1.88 | 1.94 | 4.37 | | | |
| | 3 | 983.4 | 981.7 | 2.02 | 2.02 | 2.02 | 0.00 | | | |
| a | 1 | 985.6 | 985.2 | 2.02 | 2.02 | 2.02 | 0.00 | 2.02 | 0.14 | 100 |
| | 2 | 982.4 | 982.1 | 2.02 | 2.02 | 2.02 | 0.00 | | | |
| | 3 | 984.4 | 990.2 | 2.02 | 2.03 | 2.03 | 0.35 | | | |

(2) Study of quantification of binding activity to Protein A immobilized chip

i) Evaluation of protein A-immobilized chip and study of sample concentration

Concentration of the sample anti-PTHrP antibody *e* was prepared based on the absorbance method, and then the binding concentrations of the sample *e* to the PTHrP(1-34-Cys)-immobilized chip were measured (I-(2)-i)). As a result, the binding activity value of sample *e* to PTHrP was 46%, compared to that of the standard sample *a* (Table 4). Possible reasons for these results include lowered biological activity, and contamination with foreign substances having absorbance at 280 nm. Another possible reason is that the protein content was lowered because the concentrations obtained by the absorbance method did not reflect the antibody concentration. Hence, attempts to improve the accuracy and the precision were undertaken by taking the system to quantitatively determine the bound amount to Protein A-immobilized chip as a system to quantitatively determine antibody concentration using BIACORE™, and correcting the antibody concentration.

Table 3.

| Measurement results of binding activity value of anti-PTHrP antibody *e* to PTHrP | | | | | | | | | | |
|---|---|---|---|---|---|---|---|---|---|---|
| Sample | n | Bound amount (Resonance unit, RU) | | Binding concentration calculated from calibration curve (µg/mL) | | | | Measurement (measured three times) | | Binding activity value (%) |
| | | 1st. cycle | 2nd. cycle | 1st. cycle | 2nd. cycle | Average | %cv. dupli | Average | %cv | |
| *a* | 1 | 986.7 | 1011.0 | 2.01 | 2.06 | 2.04 | 1.74 | 2.05 | 0.49 | *100* |
| | 2 | 996.1 | 1020.6 | 2.03 | 2.08 | 2.06 | 1.72 | | | |
| | 3 | 992.5 | 1009.6 | 2.03 | 2.06 | 2.05 | 1.04 | | | |
| *e* | 1 | 438.2 | 446.0 | 0.95 | 0.97 | 0.96 | 1.47 | 0.95 | 1.59 | *46.2* |
| | 2 | 428.6 | 428.6 | 0.93 | 0.93 | 0.93 | 0.00 | | | |
| | 3 | 429.4 | 435.0 | 0.94 | 0.95 | 0.95 | 0.75 | | | |

EP 1 296 140 A1

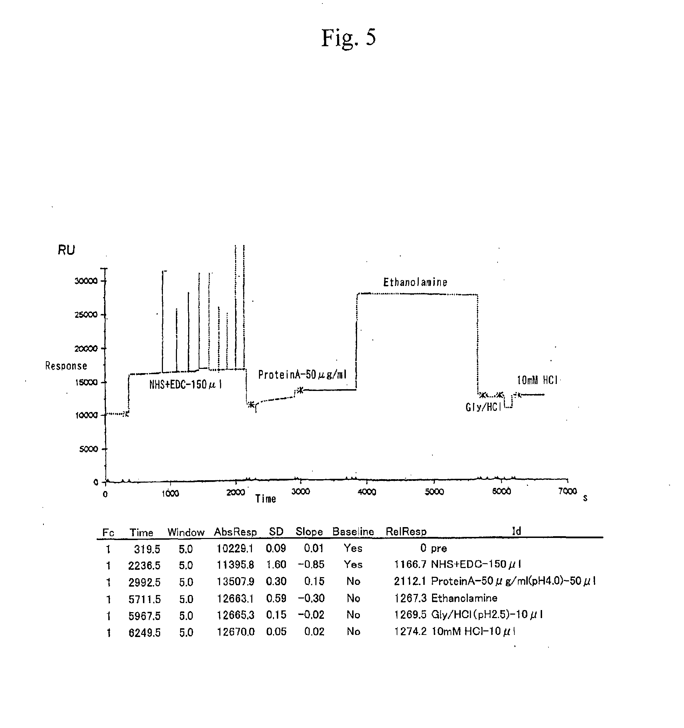

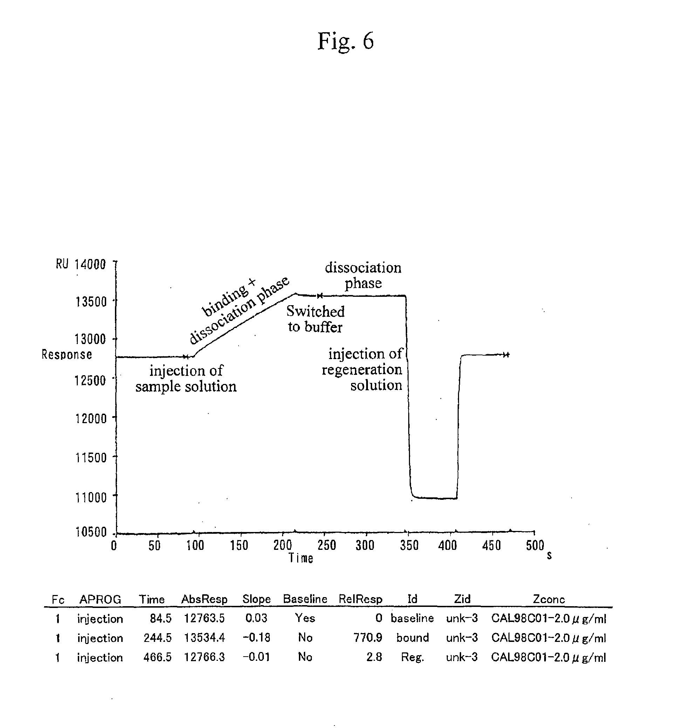

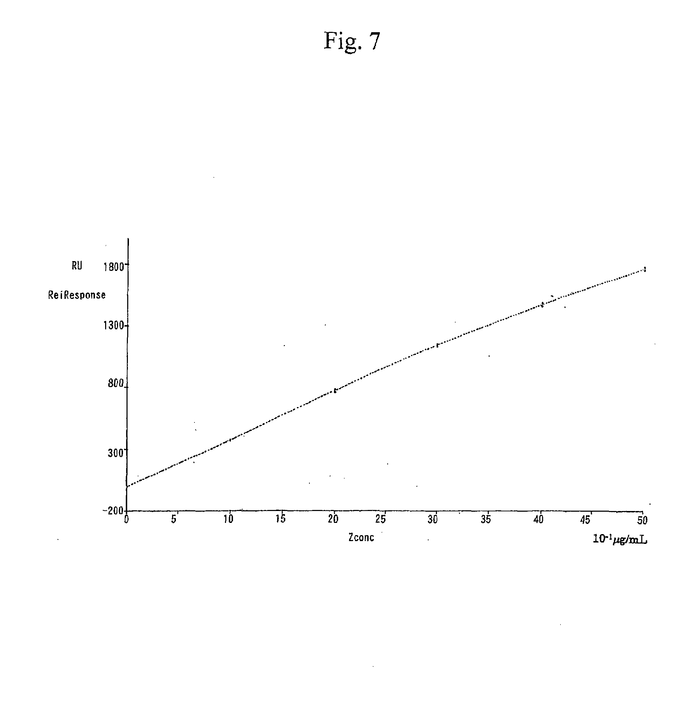

Figure 5 shows the results of the immobilization of Protein A. Based on the linearity of the sensorgram and the calibration curve (Figs. 6 and 7) resulting from the prepared Protein A-immobilized chip, it was determined that the amount of immobilized ligands was sufficient for use in the quantification system. Further, it was also confirmed that the calibration curve showed sufficient linearity with the concentration range of 0 to 5 $\mu$g/mL employed for measurement using a PTHrP(1-34-Cys)-immobilized chip. Therefore, the sample concentrations of both samples for a calibration curve and unknown samples for quantitatively determining the bound amount to the Protein A-immobilized chip were set under the same conditions with a PTHrP(1-34-Cys)-immobilized chip.

iii) Analysis of binding activity ratio of anti-PTHrP antibody *e*

Using the calibration curves for each PTHrP and Protein A, the binding concentration was calculated by means of the attached software using the bound amount provided from the sensorgram. The results were summarized in Table 5. The binding activity value to PTHrP was corrected with the concentration calculated from the bound amount to Protein A, anti-PTHrP antibody binding activity ratio was newly defined as eq. 2, and these results were summarized in Table 6. As a result, e had a binding activity of 94% when compared to a, suggesting a slightly lowered activity.

As described above, based on the quantitatively determined amount of antibodies bound to Protein A, more precise measurement of anti-PTHrP antibody binding activity became possible by the confirmation of antibody concentration using the sample concentrations, which are same as those employed for the measurement of the bound amount to PTHrP.

$$\text{Anti-PTHrP antibody binding activity ratio (BIACORE)(\%)} = \frac{\text{Binding activity value to PTHrP}}{\text{Binding activity value to Protein A}} \times 100 \quad (\text{eq. 2})$$

$$\text{Binding activity value to PTHrP} = \frac{\text{Binding concentration of unknown sample to PTHrP-immobilized chip}}{\text{Binding concentration of standard sample to PTHrP-immobilized chip}} \times 100$$

$$\text{Binding activity value to Protein A} = \frac{\text{Binding concentration of unknown sample to Protein A-immobilized chip}}{\text{Binding concentration of standard sample to Protein A-immobilized chip}} \times 100$$

Table 5.

| Measurement results of binding activity values of anti-PTHrP antibody *e* to PTHrP and Protein A | | | | | | | | | |
|---|---|---|---|---|---|---|---|---|---|
| **Ligand: PTHrP** | | | | | | | | | |
| Sample | n | Bound amount (Resonance unit, RU) | | Binding concentration calculated from calibration curve ($\mu$g/mL) | | | | Measurement (measured three times) | |
| | | 1st. cycle | 2nd. cycle | 1st. cycle | 2nd. cycle | Average | %cv. dupli | Average | %cv |

Table 5.　(continued)

| Measurement results of binding activity values of anti-PTHrP antibody *e* to PTHrP and Protein A | | | | | | | | | |
|---|---|---|---|---|---|---|---|---|---|
| **Ligand: PTHrP** | | | | | | | | | |
| Sample | n | Bound amount (Resonance unit, RU) | | Binding concentration calculated from calibration curve (μg/mL) | | | | Measurement (measured three times) | |
| *a* | 1 | 760.9 | 779.2 | 1.98 | 2.03 | 2.01 | 1.76 | 2.02 | 0.50 |
|  | 2 | 768.8 | 788.3 | 2.00 | 2.05 | 2.03 | 1.75 | | |
|  | 3 | 763.2 | 783.2 | 1.99 | 2.04 | 2.02 | 1.75 | | |
| *e* | 1 | 824.4 | 852.5 | 2.14 | 2.21 | 2.18 | 2.28 | 2.17 | 0.70 |
|  | 2 | 819.0 | 838.5 | 2.13 | 2.18 | 2.16 | 1.64 | | |
|  | 3 | 834.0 | 849.7 | 2.17 | 2.20 | 2.19 | 0.97 | | |
| **Ligand: Protein A** | | | | | | | | | |
| Sample | n | Bound amount (Resonance unit, RU) | | Binding concentration calculated from calibration curve (μg/mL) | | | | Measurement (measured three times) | |
|  |  | 1st. cycle | 2nd. cycle | 1st. cycle | 2nd. cycle | Average | %cv. dupli | Average | %cv |
| *a* | 1 | 770.5 | 786.7 | 2.00 | 2.04 | 2.02 | 1.40 | 2.03 | 0.43 |
|  | 2 | 770.9 | 785.2 | 2.00 | 2.04 | 2.02 | 1.40 | | |
|  | 3 | 776.4 | 790.4 | 2.02 | 2.05 | 2.04 | 1.04 | | |
| *e* | 1 | 875.9 | 886.0 | 2.27 | 2.30 | 2.29 | 0.93 | 2.31 | 0.99 |
|  | 2 | 876.5 | 894.0 | 2.28 | 2.32 | 2.30 | 1.23 | | |
|  | 3 | 887.6 | 909.6 | 2.30 | 2.36 | 2.33 | 1.82 | | |

Table 6.

| Measurement results of binding activity ratio of anti-PTHrP antibody *e* | | | |
|---|---|---|---|
| Sample | Binding concentration | | Binding activity ratio % |
|  | PTHrP | Protein A | |
| *a* | 2.02 | 2.03 | *100* |
| *e* | 2.17 | 2.31 | *94.7* |

(3) Estimation of repeatability of this method and measurement

To evaluate the measurement method for binding activity to PTHrP, which had been set according to the obtained results including concentration correction using Protein A, f was measured using standard product a. In one measurement, dispersions when each batch was measured twice (Dupli) were all less than 4% (83% of all measurement cycles were less than 2% dispersion), and dispersions provided when n=3 were all less than 3% (90% of all measurement cycles were less than 2% dispersion). However, dispersions resulting from one experiment repeated 7 times were less than 1% (0.95%), suggesting that the experiment system was extremely precise (Tables 7 and 8).

CAL binding activity was calculated using eq. 3 and summarized in Table 8. With the binding activity of a at 100%, the biological activity of f was at 97.0%, while the coefficient of variation of the repeatability (when repeated 7 times) was at 0.95%, these suggest that the difference between the two was significant and f had a slightly decreased activity with antigen.

Anti-PTHrP antibody binding activity ratio (BIACORE)(%)

$$= \frac{\text{Binding concentration of f to PTHrP-immobilized chip / Binding concentration of a to PTHrP-immobilized chip}}{\text{Binding concentration of f to Protein A-immobilized chip / Binding concentration of a to Protein A-immobilized chip}} \times 100 \quad \text{(eq. 3)}$$

Table 7.

| Summary of 7-times repeated measurement results of binding activity of anti-PTHrP antibody *f* | | | | | | | |
|---|---|---|---|---|---|---|---|
| N | Ligand | Sample | Mean value of binding concentration (Dupli, measured twice) (µg/mL) | | | Mean value of measurement (measured three times) | %cv |
| | | | n=1 | n=2 | n=3 | | |
| 1 | Protein A | *a* | 2.02 | 2.06 | 2.05 | 2.04 | 1.02 |
| | | *f* | 2.20 | 2.22 | 2.21 | 2.21 | 0.35 |
| | PTHrP | *a* | 2.04 | 2.05 | 2.02 | 2.04 | 0.89 |
| | | *f* | 2.14 | 2.14 | 2.12 | 2.13 | 0.41 |
| 2 | Protein A | *a* | 2.04 | 2.03 | 2.12 | 2.06 | 2.25 |
| | | *f* | 2.24 | 2.26 | 2.24 | 2.24 | 0.46 |
| | PTHrP | *a* | 2.05 | 2.05 | 2.05 | 2.05 | 0.14 |
| | | *f* | 2.17 | 2.17 | 2.20 | 2.18 | 0.80 |
| 3 | Protein A | *a* | 1.96 | 2.01 | 2.02 | 2.00 | 1.52 |
| | | *f* | 2.19 | 2.20 | 2.24 | 2.21 | 1.25 |
| | PTHrP | *a* | 2.16 | 2.19 | 2.19 | 2.18 | 0.87 |
| | | *f* | 2.33 | 2.34 | 2.39 | 2.35 | 1.29 |
| 4 | Protein A | *a* | 2.03 | 2.04 | 2.05 | 2.04 | 0.62 |
| | | *f* | 2.38 | 2.37 | 2.44 | 2.40 | 1.58 |
| | PTHrP | *a* | 1.99 | 2.05 | 2.02 | 2.02 | 1.49 |
| | | *ef* | 2.27 | 2.26 | 2.29 | 2.27 | 0.79 |
| 5 | Protein A | *a* | 2.03 | 2.02 | 2.02 | 2.02 | 0.38 |
| | | *f* | 2.44 | 2.45 | 2.39 | 2.43 | 1.29 |
| | PTHrP | *a* | 2.04 | 2.04 | 2.05 | 2.04 | 0.28 |
| | | *f* | 2.41 | 2.42 | 2.42 | 2.41 | 0.32 |
| 6 | Protein A | *a* | 1.97 | 2.02 | 2.05 | 2.01 | 2.01 |
| | | *f* | 2.73 | 2.73 | 2.78 | 2.74 | 1.00 |
| | PTHrP | *a* | 2.15 | 2.23 | 2.21 | 2.20 | 2.02 |
| | | *f* | 2.86 | 2.89 | 2.96 | 2.90 | 1.67 |

Table 7.  (continued)

| | N | Ligand | Sample | Mean value of binding concentration (Dupli, measured twice) (µg/mL) | | | Mean value of measurement (measured three times) | %cv |
|---|---|---|---|---|---|---|---|---|
| Summary of 7-times repeated measurement results of binding activity of anti-PTHrP antibody *f* | | | | | | | | |
| | | | | n=1 | n=2 | n=3 | | |
| | 7 | Protein A | *a* | 1.98 | | 1.97 | 1.98 | 0.36 |
| | | | *f* | 2.41 | 2.42 | 2.44 | 2.42 | 0.62 |
| | | PTHrP | *a* | 2.03 | 2.05 | 2.01 | 2.03 | 0.87 |
| | | | *f* | 2.38 | 2.39 | 2.42 | 2.40 | 0.75 |

Table 8.

| N | Lot No. | Binding concentration | | Binding activity ratio |
|---|---|---|---|---|
| Summary of 7-times repeated measurement results of binding activity of f | | | | |
| | | PTHrP | Protein A | % |
| 1 | *a* | 2.04 | 2.04 | 100 |
| | *f* | 2.13 | 2.21 | 96.4 |
| 2 | *a* | 2.05 | 2.06 | 100 |
| | *f* | 2.18 | 2.24 | 97.8 |
| 3 | *a* | 2.18 | 2.00 | 100 |
| | *f* | 2.35 | 2.21 | 97.6 |
| 4 | *a* | 2.02 | 2.04 | 100 |
| | *f* | 2.27 | 2.40 | 95.5 |
| 5 | *a* | 2.04 | 2.02 | 100 |
| | *f* | 2.41 | 2.43 | 98.2 |
| 6 | *a* | 2.20 | 2.01 | 100 |
| | *f* | 2.90 | 2.74 | 96.7 |
| 7 | *a* | 2.03 | 1.98 | 100 |
| | *f* | 2.40 | 2.42 | 96.7 |
| Average | *f* | - | - | 97.0 |
| 0.95 (%cv) | | | | |

$$\text{Anti-PTHrP antibody binding activity ratio (BIACORE)(\%)} = \frac{\text{Binding activity to PTHrP}}{\text{Binding activity to Protein A}} \times 100$$

$$\text{Binding activity value to PTHrP} = \frac{\text{Binding concentration of unknown sample to PTHrP-immobilized chip}}{\text{Binding concentration of standard sample to PTHrP-immobilized chip}} \times 100$$

$$\text{Binding activity value to Protein A} = \frac{\text{Binding concentration of unknown sample to Protein A-immobilized chip}}{\text{Binding concentration of standard sample to Protein A-immobilized chip}} \times 100$$

IV. Conclusion

In the measurement system, the bound amount observed at a certain time after injection of antibody followed by switching to a buffer is simply defined as the amount of PTHrP binding activity. Since the effect of dissociation is ignored in the system, the result obtained herein cannot be biological activity in the strictest sense. However, this method is good enough as a convenient method of evaluating activity without requiring kinetics analysis for samples observed from the form of the sensorgrams to have the dissociation rates sufficiently smaller than the binding rates.

As described above, we have invented a convenient and highly accurate testing method, with which in vitro CAL biological activity can be evaluated without kinetics analysis using BIACORE™. In appropriate combination with other testing methods for testing biological activity, this method can be used as a testing method which reflects in vitro environment more accurately by solving the problems inherent from the principle of BIACORE™.

All publications, patents and patent applications cited herein are incorporated herein by reference in their entirety.

Industrial Applicability

The present invention provides a convenient and highly accurate method of measuring the biological activity of a ligand.

**Claims**

1.   A method of evaluating the binding activity of a ligand to a ligand-binding protein, which comprises; measuring the binding activity value of the ligand to a ligand-binding protein, and measuring the binding activity value of the ligand to a second ligand-binding substance which binds to a site of the ligand other than the binding site where the ligand binds to the ligand-binding protein.

2.   The method of claim 1, wherein either the ligand or the ligand-binding protein is immobilized.

3.   The method of claim 1 or 2, wherein the ligand is an antigen or an antibody thereof, an enzyme or the substrate protein thereof or a ligand for various receptors.

4.   The method of claim 1 or 2, wherein the ligand is an antibody, and the ligand-binding protein is an antigen peptide.

5.   The method of claim 4, wherein the second ligand-binding substance specifically binds to the constant region or the sugar chain of an antibody.

**6.** The method of claim 5, wherein the constant region of an antibody is an Fc portion, an L chain κ region, or an L chain λ region.

**7.** The method of claim 5, wherein the second ligand-binding substance specifically binds to an Fc portion of an antibody.

**8.** The method of claim 6 or 7, wherein the substance which specifically binds to an Fc portion of an antibody is Protein A, protein G, protein L, Fc receptor or lectin.

**9.** The method of any one of claims 1 to 4, wherein FLAG protein is fused to a ligand, and a substance which specifically binds to the FLAG protein is used as the second ligand-binding protein.

**10.** The method of any one of claims 1 to 9, which comprises; separately immobilizing the ligand-binding protein and the second ligand-binding substance, allowing them to react respectively with the same ligand-containing samples, and then measuring each for binding activities.

**11.** The method of claim 4, wherein the second ligand-binding substance is Protein A.

**12.** The method of any one of claims 4 to 11, wherein the binding activity value of an antibody to an antigen peptide is corrected with the binding activity value of the antibody to the second ligand-binding substance.

**13.** The method of any one of claims 4 to 12, wherein the binding activity of an antibody to an antigen peptide is measured using the binding activity ratio of the antibody which is calculated by the following formula (I):

$$\text{Binding activity ratio (\%) of antibody} = \frac{\text{Binding activity value of antibody to antigen peptide}}{\text{Binding activity value of antibody to second ligand-binding substance}} \times 100 \quad \text{(I)}$$

**14.** The method of claim 13, wherein the binding activity value of an antibody to an antigen peptide, and the binding activity value of the antibody to the second ligand-binding substance are calculated by the following formulae (II) and (III), respectively:

$$\text{Binding activity value of antibody to antigen peptide} = \frac{\text{Binding concentration of antibody of unknown sample to antigen peptide}}{\text{Binding concentration of antibody of standard sample to antigen peptide}} \times 100 \quad \text{(II)}$$

$$\text{Binding activity value of antibody to second ligand-binding substance} = \frac{\text{Binding concentration of antibody of unknown sample to second ligand-binding substance}}{\text{Binding concentration of antibody of standard sample to second ligand-binding substance}} \times 100 \quad \text{(III)}$$

**15.** The method of any one of claims 1 to 14, wherein the binding concentration of the ligand to the ligand-binding protein, and the binding concentration of the ligand to the second ligand-binding substance are measured by either SPR, a fluorescence polarization measurement method, a radioimmunoassay (RIA), an enzyme-linked immunosorbent assay (ELISA), HPLC, an analytical ultracentrifugation method, or a titration calorimetry.

16. The method of claim 15, wherein the binding concentration of the ligand to the ligand-binding protein, and the binding concentration of the ligand to the second ligand-binding substance are measured by SPR.

17. The method of any one of claims 1 to 16, wherein the ligand is a monoclonal antibody.

18. The method of claim 17, wherein the monoclonal antibody is a humanized antibody.

19. The method of claim 17 or 18, wherein the monoclonal antibody is an antibody against parathyroid hormone-related peptide.

20. A kit for evaluating the binding activity of the ligand to the ligand-binding protein, which contains the ligand-binding protein and the second ligand-binding substance.

21. The kit of claim 20, wherein the ligand-binding protein is an antigen peptide.

# Fig. 1

| Fc | Time | Window | AbsResp | SD | Slope | Baseline | RelResp | Id |
|----|------|--------|---------|----|----|----------|---------|----|
| 2 | 476.5 | 5.0 | 10881.1 | 0.08 | −0.01 | Yes | 0 | pre |
| 2 | 1707.5 | 5.0 | 11383.3 | 1.20 | −0.61 | Yes | 702.3 | NHS+EDC−100 μl |
| 2 | 3066.5 | 5.0 | 12018.1 | 0.73 | −0.38 | No | 634.7 | PDEA−100 μl |
| 2 | 3304.5 | 5.0 | 13674.1 | 3.93 | −2.10 | No | 2290.8 | (1−34+C)10 μg/ml−10 μl−pH5.0 |
| 2 | 3721.5 | 5.0 | 13422.1 | 0.43 | −0.22 | No | 2038.7 | pre |
| 2 | 3914.5 | 5.0 | 14417.1 | 3.38 | −1.81 | No | 3033.8 | (1−34+C)10 μg/ml−10 μl−pH5.0 |
| 2 | 5423.5 | 5.0 | 12123.3 | 0.50 | −0.26 | No | 740.0 | Cys+NaCl−100 μl |
| 2 | 5677.5 | 5.0 | 12114.9 | 0.27 | −0.21 | No | 731.6 | Gly−HCl−10 μl |
| 2 | 5967.5 | 5.0 | 12102.2 | 0.19 | −0.10 | No | 718.9 | 10mM HCl−10 μl |

# Fig. 2

| Fc | APROG | Time | AbsResp | Slope | Baseline | RelResp | Id | Zid | Zconc |
|----|-------|------|---------|-------|----------|---------|-----|------|-------|
| 2 | injection | 87.5 | 12704.9 | -0.03 | Yes | 0 | baseline | unk-9 | CAL98C01-2.0 |
| 2 | injection | 247.5 | 13695.1 | 0.21 | No | 990.2 | bound | unk-9 | CAL98C01-2.0 |
| 2 | injection | 471.5 | 12715.7 | 0.02 | No | 10.8 | Reg. | unk-9 | CAL98C01-2.0 |

# Fig. 3

# Fig. 4

| Fc | APROG | Time | AbsResp | Slope | Baseline | RelResp | Id | Zid | Zconc |
|----|-------|------|---------|-------|----------|---------|----|-----|-------|
| 2 | injection | 85.5 | 11567.7 | 3.70 | Yes | 0 | baseline | 98C01 | 3.0 |
| 2 | injection | 245.5 | 13274.3 | 0.38 | No | 1706.6 | bound | 98C01 | 3.0 |
| 2 | injection | 469.5 | 12328.3 | 1.32 | No | 760.5 | Reg. | 98C01 | 3.0 |

Fig. 5

| Fc | Time | Window | AbsResp | SD | Slope | Baseline | RelResp | Id |
|----|------|--------|---------|-----|-------|----------|---------|-----|
| 1 | 319.5 | 5.0 | 10229.1 | 0.09 | 0.01 | Yes | 0 | pre |
| 1 | 2236.5 | 5.0 | 11395.8 | 1.60 | −0.85 | Yes | 1166.7 | NHS+EDC-150 $\mu$l |
| 1 | 2992.5 | 5.0 | 13507.9 | 0.30 | 0.15 | No | 2112.1 | ProteinA-50 $\mu$g/ml(pH4.0)-50 $\mu$l |
| 1 | 5711.5 | 5.0 | 12663.1 | 0.59 | −0.30 | No | 1267.3 | Ethanolamine |
| 1 | 5967.5 | 5.0 | 12665.3 | 0.15 | −0.02 | No | 1269.5 | Gly/HCl(pH2.5)-10 $\mu$l |
| 1 | 6249.5 | 5.0 | 12670.0 | 0.05 | 0.02 | No | 1274.2 | 10mM HCl-10 $\mu$l |

EP 1 296 140 A1

Fig. 6

| Fc | APROG | Time | AbsResp | Slope | Baseline | RelResp | Id | Zid | Zconc |
|---|---|---|---|---|---|---|---|---|---|
| 1 | injection | 84.5 | 12763.5 | 0.03 | Yes | 0 | baseline | unk-3 | CAL98C01-2.0 $\mu$ g/ml |
| 1 | injection | 244.5 | 13534.4 | -0.18 | No | 770.9 | bound | unk-3 | CAL98C01-2.0 $\mu$ g/ml |
| 1 | injection | 466.5 | 12766.3 | -0.01 | No | 2.8 | Reg. | unk-3 | CAL98C01-2.0 $\mu$ g/ml |

Fig. 7

## INTERNATIONAL SEARCH REPORT

| | International application No. |
|---|---|
| | PCT/JP01/05501 |

| A. CLASSIFICATION OF SUBJECT MATTER |
|---|
| Int.Cl⁷ G01N33/53, G01N33/543, G01N33/566 |

According to International Patent Classification (IPC) or to both national classification and IPC

| B. FIELDS SEARCHED |
|---|
| Minimum documentation searched (classification system followed by classification symbols) |
| Int.Cl⁷ G01N33/53, G01N33/543, G01N33/566 |

Documentation searched other than minimum documentation to the extent that such documents are included in the fields searched
Jitsuyo Shinan Koho        1922-1996    Toroku Jitsuyo Shinan Koho  1994-2001
Kokai Jitsuyo Shinan Koho  1971-2001    Jitsuyo Shinan Toroku Koho   1996-2001

Electronic data base consulted during the international search (name of data base and, where practicable, search terms used)

| C. DOCUMENTS CONSIDERED TO BE RELEVANT | | |
|---|---|---|
| Category* | Citation of document, with indication, where appropriate, of the relevant passages | Relevant to claim No. |
| A | JP 9-504859 A (Pharmacia Biosensor AB), 13 May, 1997 (13.05.97), & WO 94/28416 A  & SE 700519 A & US 5753518 A  & DE 69426155 E & EP 700519 B | 1-21 |
| A | WO 97/45746 A (McGill University), 04 December, 1997 (04.12.97), & AU 9745746 A  & EP 920630 A & BR 9709900 A  & JP 2000-512131 A | 1-21 |

☐ Further documents are listed in the continuation of Box C.    ☐ See patent family annex.

| * | Special categories of cited documents: |
|---|---|
| "A" | document defining the general state of the art which is not considered to be of particular relevance |
| "E" | earlier document but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 13 August, 2001 (13.08.01) | 28 August, 2001 (28.08.01) |

| Name and mailing address of the ISA/ | Authorized officer |
|---|---|
| Japanese Patent Office | |
| Facsimile No. | Telephone No. |

Form PCT/ISA/210 (second sheet) (July 1992)